# EUROPEAN PATENT APPLICATION

(11) **EP 1 654 991 A1**
(43) Date of publication of application: **10.05.2006**
(21) Application number: 04026485.5
(22) Date of filing: 08.11.2004
(51) Int. Cl.: A61B 17/12

(54) **Screw for vascular occlusion**

(71) Applicant: Inderbitzi, Rolf, Dr. med., 8967 Widen (CH)
(72) Inventor: Inderbitzi, Rolf, Dr. med., 8967 Widen (CH)
(74) Representative: Heinze, Ekkehard

(57) **Abstract**

A vascular occlusion screw (100) and an insertion instrument (200) are provided for occluding bleeding vessels during surgical procedures. The vascular occlusion screw has a screw head (102) and a body (104). The body has a threaded surface (108) and a pointed tip (110). The threaded surface and the pointed tip help to fix the vascular occlusion screw inside the lumen of a blood vessel. The insertion instrument has a handgrip (202), a head (204), a shaft (206), and a cover (208). The insertion instrument helps to insert the vascular occlusion screw into the lumen blood vessel.

## Description

### BACKGROUND

The present invention relates to the field of medical devices. In particular, the present invention relates to an implantable device used for occluding blood vessels during surgical procedures.

Occlusion of blood vessels is required to control blood loss during surgical procedures. One example of such a surgical procedure is the surgical treatment of an abdominal aortic aneurysm.

Abdominal aortic aneurysms are dilatations of the abdominal aorta that result due to the weakening of the wall of the abdominal aorta. Aneurysms of the abdominal aorta may lead to complications such as the spontaneous rupture of the wall of the abdominal aorta. The risk of spontaneous rupture increases when the transverse size of the aneurysm becomes more than 5 cm.

The spontaneous rupture of an abdominal aortic aneurysm leads to massive internal bleeding, which can prove to be fatal within minutes. Other problems that may be caused by abdominal aortic aneurysms include emboli to the lower limbs and compression of adjacent structures such as abdominal viscera, arteries, veins, and nerves.

In order to avoid the risk of spontaneous rupture and other complications associated with abdominal aortic aneurysms, surgical repair of abdominal aortic aneurysms is indicated.

Surgical treatment of the abdominal aortic aneurysms involves the replacement of the diseased part of the abdominal aorta by a vascular prosthesis. This procedure is conducted either through an open surgical procedure or by laparoscopic surgery. During this procedure, the abdominal aorta is clamped above and below the aneurysmal section. The wall of the aneurysm is then incised. Once the wall of the aneurysm is incised, the arteries that originate from the aneurysmal section are exposed and start to bleed profusely. If these vessels are not occluded, excessive loss of blood may occur. Excessive blood loss may lead to complications during and after the surgical procedure.

Various methods are employed to stop the flow of blood from the exposed blood vessels during the surgical procedure for repairing the abdominal aortic aneurysm. These methods involve the application of external pressure on the bleeding vessels, or the insertion of occluding devices into the lumen of the blood vessels. External pressure may be applied on the blood vessel by a surgeon's fingers, by ligating the blood vessel by means of a suture, or by the application of occluding devices such as forceps or clips.

Application of external pressure manually by the surgeon's or the surgeon's assistant's fingers is suitable for temporary occlusion of the bleeding blood vessels. However, it cannot be used for permanent occlusion. Moreover, the application of manual pressure interferes with the operative space and may cause hindrance to effectively carry out the surgical procedure. Another limitation of this technique is that it cannot be employed during a laparoscopic procedure.

During the ligation of the bleeding blood vessels by a suture, the surgeon identifies the bleeding blood vessel and ties a suture around it, thereby occluding it. However, the application of sutures to blood vessels is sometimes difficult, especially during laparoscopic procedures. Moreover, at times, the application of sutures may not lead to a secure occlusion, since the ligature may slip from the blood vessel. In the case of atherosclerotic blood vessels, the ligature may not completely occlude the lumen of the blood vessel. Another problem with the application of sutures on the atherosclerotic blood vessel wall is that the suture may further damage the blood vessel. A tightly applied suture may break through the wall of the blood vessel and result in bleeding.

Occluding devices such as forceps or clips, which apply external pressure on blood vessels, are used to occlude bleeding blood vessels. However, application of external pressure by means of forceps and clips interferes with the operative field. Furthermore, these cannot be applied to bleeding vessels located behind the wall of the aneurysm. Moreover, it is difficult to use these devices during a laparoscopic procedure since they interfere with an already crowded operative field. In addition, these devices may not result in complete occlusion of the lumen of the bleeding blood vessels, especially in the case of atherosclerotic blood vessels.

In light of the above discussion, there is a need for an implantable device that quickly and effectively occludes a blood vessel during surgical procedures. The device should reduce the amount of blood loss during a surgical procedure. It should be useful during open surgical procedures as well as laparoscopic surgeries. Moreover, such a device should not interfere with access to the operative field. Further, it should help to safely occlude anatomically hidden vessels.

### SUMMARY

An object of the invention is to provide a vascular occlusion screw that is suitable for occluding bleeding vessels during surgical procedures.

Another object of the present invention is to provide a system suitable for occluding bleeding vessels during laparoscopic surgery.

Yet another object of the present invention is to provide a system suitable for safely occluding anatomically hidden vessels.

Yet another object of the present invention is to provide a system suitable for occluding bleeding blood vessels without interfering with the operating field.

Still another object of the present invention is to provide an insertion instrument to insert the vascular occlusion screw into the lumen of the bleeding vessels.

In accordance with an exemplary embodiment of the present invention, a vascular occlusion screw and an insertion instrument are provided for occluding bleeding blood vessels. The vascular occlusion screw comprises a screw head and a body. The screw head has a groove, to attach to the insertion instrument. The body has a threaded surface and a pointed tip. The threaded surface helps to occlude the lumen of the bleeding vessel; the pointed tip helps to insert the vascular occlusion screw into the lumen of the bleeding blood vessel.

In accordance with an exemplary embodiment of the present invention, the insertion instrument comprises a handgrip, a head, a shaft, and a cover. The cover helps to protect the vascular occlusion screw while the vascular inclusion screw is inserted during laparoscopic surgery.

In accordance with an exemplary embodiment of the present invention, a method is presented for occluding bleeding blood vessels during surgical procedures, using a vascular occlusion screw and an insertion instrument. After attaching the vascular occlusion screw to the insertion instrument, the vascular occlusion screw is driven into the lumen of the bleeding blood vessel by applying force through the insertion instrument.

The additional objects and advantages of the invention will become apparent to those skilled in the art, by referring to the following detailed description, in conjunction with the figures provided.

### BRIEF DESCRIPTION OF THE DRAWINGS

The various embodiments of the invention will hereinafter be described in conjunction with the appended drawings provided to illustrate and not to limit the invention, wherein like designations denote like elements, and in which:
FIG. 1 is a representation of a vascular occlusion screw (100), in accordance with an embodiment of the present invention.
FIG. 2 is a representation of an insertion instrument (200) for inserting the vascular occlusion screw, in accordance with an embodiment of the present invention.
FIG. 3 is a flowchart of a method for occluding a bleeding blood vessel using the vascular occlusion screw during open surgical procedures, in accordance with an embodiment of the present invention.
FIG. 4 is a flowchart of a method for occluding a bleeding blood vessel using the vascular occlusion screw during laparoscopic surgery, in accordance with an embodiment of the present invention.
FIG. 5 is a representation of the completed procedure for occluding a bleeding blood vessel using the vascular occlusion screw, in accordance with an embodiment of the present invention.

### DETAILED DESCRIPTION OF THE INVENTION

The present invention discloses a Vascular Occlusion Screw System (VOSS) for the occlusion of blood vessels during surgical procedures. In particular, the invention provides a vascular occlusion screw for the occlusion of bleeding spinal and lumbar arteries during a surgical procedure for the repair of an abdominal aortic aneurysm. The vascular occlusion screw has a threaded body that helps in occluding the lumen of the blood vessels securely. The present invention further provides an insertion instrument for inserting the vascular occlusion screw into the lumen of a blood vessel.

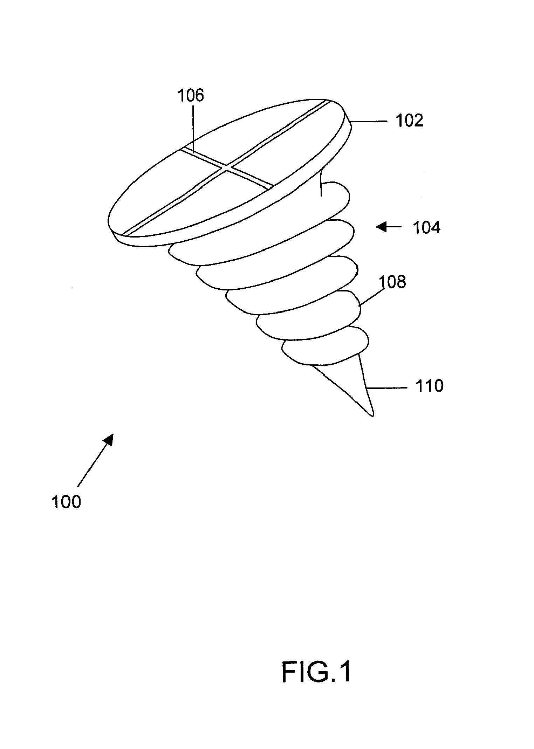

FIG. 1 is a representation of vascular occlusion screw 100, in accordance with an embodiment of the present invention. Vascular occlusion screw 100 has a screw head 102 and a body 104. Force is applied to screw head 102, to fix vascular occlusion screw 100 inside the lumen of a blood vessel. Screw head 102 has a groove 106 on one surface. In accordance with an embodiment of the present invention, groove 106 is a cross groove. Body 104 has a threaded surface 108 and a tip 110.

In accordance with an embodiment of the present invention, the diameter of screw head 102 is within a range of 3 mm to 6 mm, the preferred diameter being 4 mm. In accordance with an embodiment of the present invention, the thickness of screw head 102 is within a range of 1 mm to 3 mm, the preferred thickness being 2 mm. In accordance with an embodiment of the present invention, the depth of groove 106 on screw head 102 is within a range of 0.5 mm to 1.5 mm, the preferred depth being 1 mm.

In accordance with an embodiment of the present invention, the length of body 104 is within a range of 2 mm to 10 mm, the preferred length being 4 mm.

In accordance with an embodiment of the present invention, diameter of body 104 tapers from screw head 102 towards tip 110. Body 104 is attached to screw head 102 at its maximum diameter, and the minimum diameter of body 104 is at tip 110. In accordance with an embodiment of the present invention, the maximum diameter of body 104 is within a range of 2 mm to 6 mm, the preferred maximum diameter being 3.5 mm. In accordance with an embodiment of the present invention, the minimum diameter of body is within a range of 0.5 mm to 2 mm, the preferred minimum diameter being 1 mm.

In accordance with an embodiment of the present invention, vascular occlusion screw 100 is made of an absorbable biocompatible material. In accordance with an embodiment of the present invention, the absorbable biocompatible material is Polydioxanone. In accordance with an embodiment of the present invention, vascular occlusion screw 100 is made of a non-absorbable biocompatible material. In accordance with an embodiment of the present invention, the non-absorbable biocompatible material is Polydioxanone.

Vascular occlusion screw 100 is inserted into the lumen of the blood vessel by means of an instrument such as the Philips Screw Driver (known in the art).

However, during laparoscopic surgery, vascular occlusion screw 100 has to be inserted through small incisions in the abdominal wall. In such cases, it becomes extremely important that vascular occlusion screw 100 is securely attached to the instrument used to insert it.

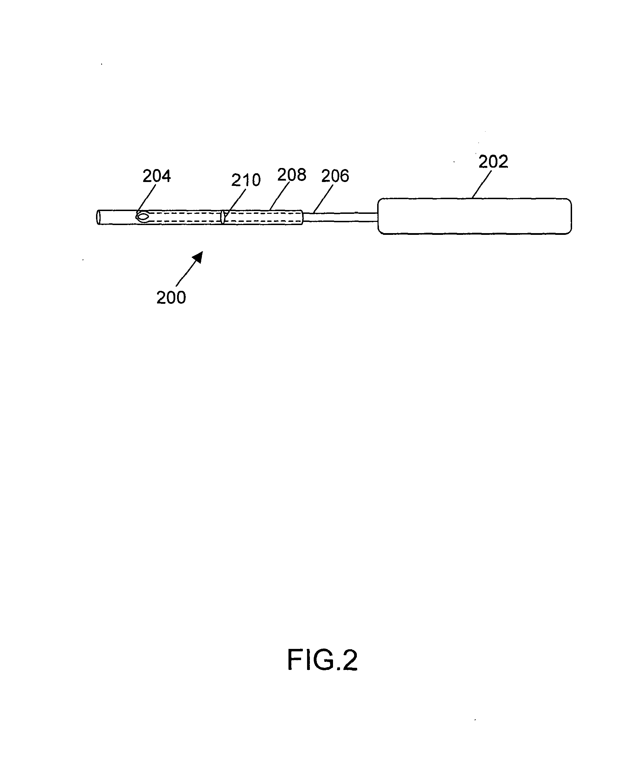

FIG. 2 is a representation of insertion instrument 200 for inserting vascular occlusion screw 100 during laparoscopic surgery, in accordance with an embodiment of the present invention. Insertion instrument 200 has a handgrip 202, a head 204, a shaft 206, and a cover 208. Handgrip 202 helps in holding insertion instrument 200, and in applying force to insertion instrument 200. Head 204 helps in attaching insertion instrument 200 to groove 106 of screw head 102, and in transmitting force to screw head 102. Shaft 206 helps in transmitting force from handgrip 202 to head 204. Cover 208 can be retracted over shaft 206 to expose head 204.

The attachment of vascular occlusion screw 100 to head 204 is especially important in the case of laparoscopic surgery. Cover 208 protects vascular occlusion screw 100 while it is being inserted during laparoscopic surgery. Insertion instrument 200, with cover 208, can be inserted into an access port created for laparoscopic surgery in a body cavity. In accordance with an embodiment of the present invention, cover 208 has a valve 210, which prevents the passage of body fluids from inside a body cavity to the outside during a surgical procedure. In addition, valve 210 prevents the passage of air from outside into a body cavity during a laparoscopic surgery.

In accordance with an embodiment of the present invention, the length of handgrip 202 is within a range of 100 mm to 160 mm, the preferred length being 100 mm. In accordance with an embodiment of the present invention, the diameter of handgrip 202 is within a range of 30 mm to 50 mm, the preferred diameter being 40 mm. In accordance with an embodiment of the present invention, the length of head 204 is within a range of 10 mm to 30 mm, the preferred length being 20 mm. In accordance with an embodiment of the present invention, the diameter of head 204 is within a range of 3 mm to 7 mm, the preferred diameter being 6 mm. In accordance with an embodiment of the present invention, the length of shaft 206 is within a range of 100 mm to 200 mm, the preferred length being 140 mm. In accordance with an embodiment of the present invention, the diameter of shaft 206 is within a range of 3 mm to 10 mm, the preferred diameter being 5 mm.

In accordance with an embodiment of the present invention, handgrip 202 is made of aluminum. In accordance with an embodiment of the present invention, head 204 is made of biocompatible material. In accordance with an embodiment of the present invention, the biocompatible material is Titan. In accordance with an embodiment of the present invention, shaft 206 is made of biocompatible material. In accordance with an embodiment of the present invention, the biocompatible material is Titan.

In accordance with an embodiment of the present invention, cover 208 is made of biocompatible material. In accordance with an embodiment of the present invention, the biocompatible material is Titan. In accordance with an embodiment of the present invention, valve 210 is made of a biocompatible material. In accordance with an embodiment of the present invention, the biocompatible material is silicone.

Having described vascular occlusion screw 100, and insertion instrument 200 for fixing vascular occlusion screw 100 inside a blood vessel, a method for occluding a bleeding blood vessel using these is described hereinafter. As an example of occluding a bleeding blood vessel during surgical procedures, a procedure for occluding bleeding blood vessels during a surgery for repair of an abdominal aortic aneurysm is described. The bleeding blood vessels are spinal arteries, lumbar arteries, or inferior mesenteric arteries that originate from the diseased aneurysmal section of the abdominal aorta.

During surgery for the repair of an abdominal aortic aneurysm, the diseased aneurysmal section of the aorta is excluded from the circulation of blood by applying vascular clamps. These clamps are applied proximal and distal to the diseased section of the aorta. Vascular clamps such as the Fogarty aortic clamp may be used for this purpose. After applying the vascular clamps, the diseased aneurysmal section of the aorta is transected. During this procedure, arteries such as spinal arteries, lumbar arteries, or the inferior mesenteric artery are exposed and they may start bleeding. This bleeding is due to the back flow of blood from these blood vessels because of collateral circulation. Vascular occlusion screw 100 is used to occlude these bleeding vessels.

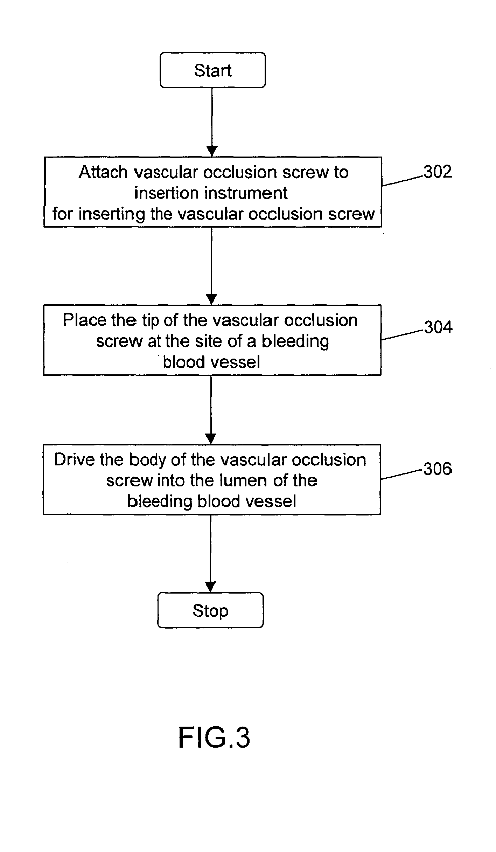

FIG. 3 is a flowchart of a method for occluding a bleeding blood vessel, using vascular occlusion screw 100, during open surgical procedures, in accordance with an embodiment of the present invention. At step 302, vascular occlusion screw 100 is attached to an insertion instrument such as the Philips Screw Driver for inserting vascular occlusion screw 100. Vascular occlusion screw 100 is attached to the insertion instrument by fixing the insertion instrument to groove 106. At step 304, tip 110 of vascular occlusion screw 100 is placed at the site of the bleeding blood vessel, such as the spinal, lumbar or inferior mesenteric arteries. At step 306, body 104 of vascular occlusion screw 100 is driven into the lumen of the bleeding blood vessel by applying force through the insertion instrument. Body 104 of vascular occlusion screw 100 occludes the lumen of the bleeding blood vessel and stops back bleeding.

The bleeding blood vessels lying behind the wall of the transected section of the abdominal aortic aneurysm are also occluded using vascular occlusion screw 100. Use of vascular occlusion screw 100 results in decreased blood loss by occlusion of bleeding blood vessels without obstructing the filed of operation. Also, anatomically hidden blood vessels are occluded using vascular occlusion screw 100.

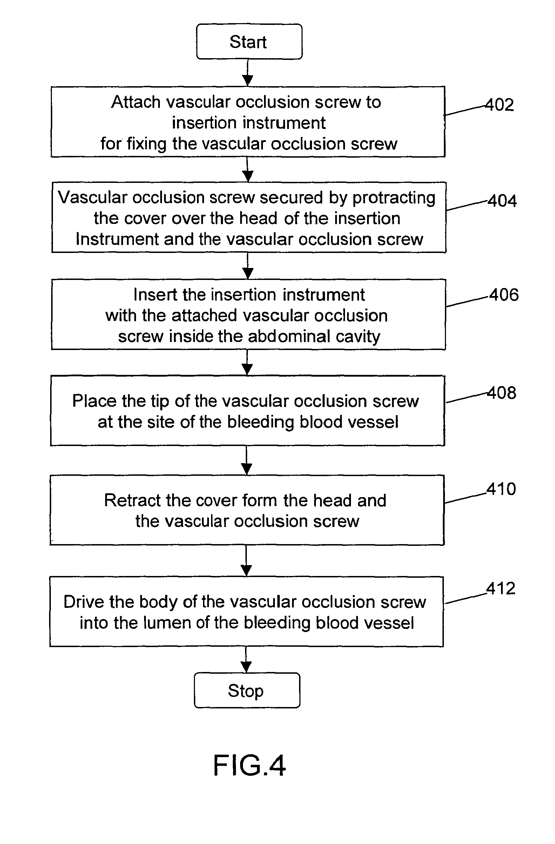

Vascular screw system 100, along with insertion instrument 200, can be used to occlude bleeding blood vessels during laparoscopic surgery for repair of abdominal aortic aneurysms. FIG. 4 is a flowchart illustrating a method for occluding a bleeding blood vessel, using vascular occlusion screw 100 along with insertion instrument 200, during laparoscopic surgery for repair of abdominal aortic aneurysms, in accordance with an embodiment of the present invention. At step 402, vascular occlusion screw 100 is attached to head 204 of insertion instrument 200. Vascular occlusion screw 100 is attached to insertion instrument 200 by fixing head 204 to groove 106. At step 404, cover 208 is protracted to secure vascular occlusion screw 100 to head 204. At step 406, insertion instrument 200, along with attached vascular occlusion screw 100, is inserted into the abdominal cavity. At step 408, tip 110 of vascular occlusion screw 100 is placed at the site of a bleeding blood vessel, such as spinal, lumbar or inferior mesenteric arteries. At step 410, cover 208 is retracted from head 204 and attached to vascular occlusion screw 100. At step 412, body 104 is driven into the lumen of the bleeding blood vessel by applying force to handgrip 202. Body 104, when in place, occludes the lumen of the bleeding blood vessel and stops the back flow of blood from the blood vessel.

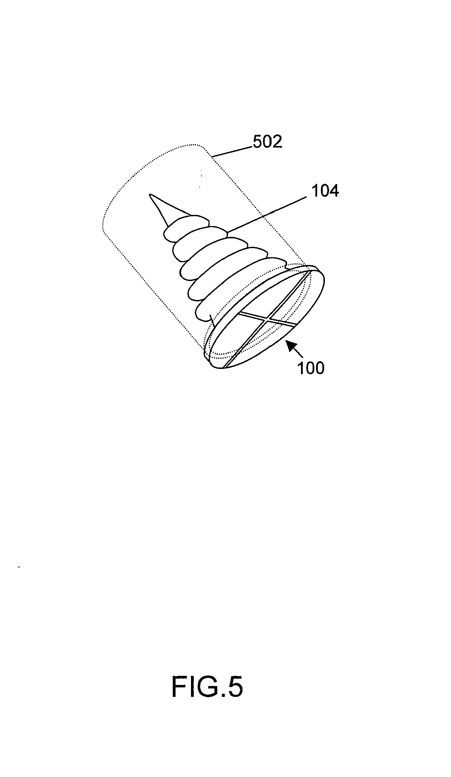

FIG. 5 is a representation of the completed procedure for occluding a bleeding blood vessel, in accordance with an embodiment of the present invention. Body 104 of vascular occlusion screw 100 is inside the lumen of blood vessel 502 and is occluding the flow of blood.

While the various embodiments of the invention have been illustrated and described, it will be clear that the invention is not limited to these embodiments only. Numerous modifications, changes, variations, substitutions and equivalents will be apparent to those skilled in the art without departing from the spirit and scope of the invention as described in the claims.

## Claims

1. A vascular occlusion screw (100) for occlusion of a blood vessel, the vascular occlusion screw comprising:
a. a screw head (102), the screw head helping to fix the vascular occlusion screw inside the lumen of the blood vessel; and
b. a body (104), the body having a threaded surface, the threaded surface helping to occlude the lumen of the of the blood vessel.

2. The vascular occlusion screw of claim 1 wherein the screw head comprises at least one groove (106) for attachment with an insertion instrument.

3. The vascular occlusion screw of claim 1 or 2 wherein the vascular occlusion screw comprises an absorbable biocompatible material.

4. The vascular occlusion screw of one of claims 1 to 3 wherein the vascular occlusion screw comprises a non-absorbable biocompatible material.

5. A vascular occlusion screw system for occluding a blood vessel, the vascular occlusion screw system comprising:
a. A vascular occlusion screw (100), the vascular occlusion screw comprising:
i. a screw head (102), the screw head helping to fix the vascular occlusion screw inside the lumen of the blood vessel; and
ii. a body (104), the body having a threaded surface (108), the threaded surface helping to occlude the lumen of the blood vessel;
b. an insertion instrument (200) for inserting the vascular occlusion screw into the lumen of a blood vessel, the insertion instrument being adapted to the occlusion screw.

6. The vascular occlusion screw system of claim 5, wherein the insertion instrument comprises:
i. a handgrip (202), the handgrip helping in holding the insertion instrument and applying force to insert the vascular occlusion screw;
ii. a head (204), the head helping in inserting the vascular occlusion screw into the lumen of the blood vessel;
iii. a shaft (206), the shaft connecting the head to the handgrip and helping to transmit the force from the handgrip to the head; and
iv. a cover (208), the cover protecting the vascular occlusion screw during the insertion of the vascular occlusion screw into the lumen of the blood vessel.

7. The vascular occlusion screw system of claim 5 or 6, wherein the screw head comprises at least one groove (106) for attachment with the insertion instrument;

8. The vascular occlusion screw system of one of the claims 5-7, wherein the vascular occlusion screw comprises an absorbable biocompatible material.

9. The vascular occlusion screw system of one of the claims 5 to 8, wherein the vascular occlusion screw comprises a non-absorbable biocompatible material.
